# EUROPÄISCHE PATENTSCHRIFT

(11) **EP 1 750 599 B1**
(45) Veröffentlichungstag und Bekanntmachung des Hinweises auf die Patenterteilung: **19.05.2010**
(21) Anmeldenummer: 05741816.2
(22) Anmeldetag: 12.05.2005
(51) Int. Cl.: A61B 17/28

(54) **GRIFFTEIL FÜR EIN CHIRURGISCHES INSTRUMENT**
GRIP ELEMENT FOR A SURGICAL INSTRUMENT
PARTIE DE PREHENSION D'UN INSTRUMENT CHIRURGICAL

(30) Priorität: 18.05.2004 DE 102004024658; 22.06.2004 DE 102004030030
(43) Veröffentlichungstag der Anmeldung: 14.02.2007
(73) Patentinhaber: Erbe Elektromedizin GmbH, 72072 Tübingen (DE)
(72) Erfinder: HILLER, Jürgen, 72581 Dettingen (DE)
(74) Vertreter: Bohnenberger, Johannes
(86) Internationale Anmeldenummer: PCT/EP2005/005197
(87) Internationale Veröffentlichungsnummer: WO 2005/112795

(56) Entgegenhaltungen:
- WO-A-99/05975
- WO-A-03/101316
- US-A- 5 782 749
- US-A- 5 908 436
- US-B2- 6 540 737

## Beschreibung

Die Erfindung betrifft ein Griffteil für ein chirurgisches Instrument, insbesondere für ein chirurgisches Rohrschaftinstrument. Das Griffteil weist einen Handgriff und eine an das chirurgische Instrument angepasste Instrumentenhalterung auf, wobei der Handgriff relativ zu der Instrumentenhalterung drehbar und/oder verschiebbar ist.

Griffteile dieser Art werden allgemein verwendet, um chirurgische Instrumente, insbesondere chirurgische Rohrschaftinstrumente, zu halten. Ferner ist bekannt, Griffteile derart auszubilden, dass das chirurgische Instrument über das Griffteil bedienbar ist.

Rohrschaftinstrumente sind spezielle chirurgische Instrumente, die insbesondere in der endoskopischen Chirurgie Anwendung finden. Sie sind angepasst, durch eine enge Körperöffnung hindurch zu einer Zielstelle innerhalb der Körperhöhle geführt zu werden und an der Zielstelle eine gewünschte Aktion, wie beispielsweise Abklemmen, Schneiden, Entnehmen einer Gewebeprobe oder Stillen von Blutungen, durchzuführen. Dieser Anwendung entsprechend, ist ein längliches Zwischenstück zwischen einem Werkzeug und dem Griffteil angeordnet. Über das Griffteil ist das Rohrschaftinstrument positionierbar und bedienbar. Ferner ist bekannt, an dem Griffteil elektrische Anschlüsse anzubringen und entsprechende Leitungen durch das Zwischenstück zu führen, um an dem Werkzeug eine Spannung, insbesondere eine hochfrequente Wechselspannung, anzulegen.

Die nachfolgend genannten Dokumente zeigen, dass eine Vielzahl verschiedener chirurgischer Instrumente bekannt ist. Die jeweilige Ausführung eines Werkzeuges, eines Zwischenstücks, elektrischer Anschlüsse sowie einer mechanischen Kopplung zwischen einem Bedienelement am Griffteil und dem Werkzeug sind nicht Gegenstand der vorliegenden Erfindung. Dementsprechend wird von einer detaillierten Diskussion dieser Bauteile abgesehen.

US 5 250 073 betrifft ein Rohrschaftinstrument in Form einer Biopsiezange, welche einen Griff, einen länglichen Hohlkörper und am distalen Ende des Rohtschaftinsttuments ein Werkzeug in Form von Zangenbacken aufweist Der längliche Hohlkörper erstreckt sich zwischen einem distalen Ende des Griffs und den Zangenbacken. Ein weiteres Rohrschaftinstrument mit einem Griff ist aus der WO 94 05223 bekannt Die Anordnung des Griffs relativ zu den restlichen Teilen des Rohrschaftinstruments ist sowohl bei der US 5 250 073 als auch bei der WO 94 05223 unveränderlich.

Weitere Rohrschaftinstrumente mit fest angeordneten Griffen sind aus den US 5 569 243, US 5 391 166 und US 5 545170 bekannt

US 5 258 006 beschreibt ein bipolares Rohrschaftinstrument mit einem Griff, einem Werkzeug in Form einer Zange am distalen Ende des Rohrschaftinstruments, einem äußeren Rohrstück und einem inneren Rohrstück, das innerhalb des äußeren Rohrstücks angeordnet ist Der Griff ist relativ zu dem äußeren Rohrstück ortsfest angeordnet Durch Betätigen eines Betätigungselements, das vorzugsweise als Schwenkhebel ausgebildet ist, wird das innere Rohrstück relativ zu dem äußeren Rohrstück verschoben, so dass ein Öffnen bzw. ein Schließen der Zangenbacken bewirkt wird. Ferner sind elektrische Leitungen vorgesehen, welche durch das innere Rohrstück durchgeführt sind, und über welche zwischen den beiden Zangenbacken eine Wechselspannung anlegbar ist Das Rohrschaftinstrument weist Mittel auf, mittels welcher das äußere und das innere Rohrstück zusammen mit den elektrischen Leitungen um eine Drehachse, die parallel zu der Längsachse des Rohrschaftinstruments verläuft, drehbar sind. Eine solche Drehbewegung wird vorzugsweise über einen Drehknopf, der am Griff angeordnet ist, ausgelöst

Eine Vorrichtung zum Entnehmen von Gewebeproben aus tiefer gelegenen Körperstellen ist aus der WO 96 06563 bekannt Ein flexibler Katheterabschnitt ist zwischen einem Greifteil und einem Schneidewerkzeug angeordnet, wobei der Katheterabschnitt mit dem vorderseitig angeordneten Schneidewerkzeug durch eine geeignete röhrenartige Hülle, beispielsweise durch eine Vene, in den Körper einführbar ist, so dass das Schneidewerkzeug an die gewünschte Körperstelle zum Entnehmen der Gewebeprobe positionierbar ist Ferner wird über das Greifteil ein Zuschnappen des Schneidewerkzeuges ausgelöst Aufgrund des flexiblen Katheterabschnitts ist eine bequeme Handstellung beim Bedienen möglich, eine stabile Anordnung zwischen Greifteil und Schneidewerkzeug kann jedoch nicht erreicht werden.

Ferner ist in US 2 427 873 eine Vorrichtung zum Einsetzen von Klammern beschrieben, welche zum Verschließen von Hautschnitten, offenen Arterien o.ä. verwendbar ist Die Vorrichtung weist ein Greif- und Bedienteil auf, das über ein rohrförmiges Zwischenstück mit dem eigentlichen Werkzeug zum Einsetzen der Klammern verbunden ist. Das Rohrstück mitsamt dem Werkzeugende ist von dem Greif- und Bedienteil abnehmbar, und kann ferner um eine Drehachse, die parallel zu seiner Längsachse verläuft, gedreht und in einer gewünschten Ausrichtung an dem Greif- und Bedienteil arretiert werden.

DE 41 32 261 beschreibt einen ergonomischen Griff für chirurgische Instrumente, bei welchem zwei Griffteile in zwei Augen für Zeige- und Mittelfinger enden und scherenartig relativ zueinander bewegbar sind. Ferner ist am oberen Griffteil eine Pfanne als Anlagefläche für den Daumen angebracht.

Aus der DE 197 02 447 C2 ist ein Instrumentengriff für ein chirurgisches Rohrschaftinstrument bekannt. Das Griffteil umfasst einen Schafthalter mit einem Schaftaufnahmeteil für den Rohrschaft des Rohrschaftinstruments und einem schräg zur Längsachse des Rohrschafts ausgerichteten Trägerelement. Ein Griff steht von dem proximalen Endbereich des Trägerelements schräg ab, kreuzt die Längsachse des Rohrschaftes und erstreckt sich über diese hinaus. Ferner ist ein Betätigungselement, vorzugsweise in Form eines Schwenkhebels, vorgesehen, welches zum Betätigen eines am distalen Ende des Rohrschafts angeordneten Werkzeugs verschwenkbar am Griffteil gelagert ist. Durch eine derartige Konstruktion soll es dem Chirurgen ermöglicht werden, beim Halten des Instrumentengriffs seinen Unterarm in eine mit der Längsachse des Rohrschafts fluchtende Stellung zu bringen.

US 5,782,749 A zeigt ein gattungsgemäßes Instrument mit einem relativ zum Instrumentenkörper bewegbaren Handgriffteil. Dieser Handgriffteil kann dabei Richtung distal-proximal verschoben oder um die Längsachse des Rohrschafts gedreht werden, um dem Anwender eine möglichst an seine Hand angepasste Stellung zu ermöglichen. Auch hier hat sich jedoch gezeigt, dass diese Anpassung unzureichend ist.

US 5,908,436 A zeigt die Drehbarkeit in zwei Ebenen eines Handgriffs relativ zum distalen Ende an einem gattungsgemäßen Instrument. Zwar steht auch hier die Ergonomie als Aufgabe im Raum, um das Handgelenk zu entlasten, hier ist die Drehbarkeit mitunter jedoch gleichzeitig das Mittel, um das Instrument auch zu bedienen. Der Handgriff kann dabei um eine Achse gedreht werden. Diese Achse ist relativ zum Handgriff um eine gewisse Distanz verschoben, der Handgriff dreht sich also nicht um sich selbst, sondern tritt mit der Drehung gleichzeitig aus der Handgriffebene heraus. Hinsichtlich der Adaptierbarkeit an den jeweiligen Anwender während der Operation besteht ebenfalls Verbesserungsbedarf.

Die WO 03/101316 A1 zeigt ein medizinisches Instrument, insbesondere für endoskopische Eingriffe, mit einem Instrumentenschaft, einen am distalen Ende des Instrumentenschaftes angeordneten Werkzeug sowie einer Handhabe, die über ein Kopplungselement in axialer Erstreckung des Instrumentenschaftes verlagerbar am Instrumentenschaft festlegbar ist. Um ein medizinisches Instrument zu schaffen, das auch bei den unterschiedlichsten Verwendungszwecken eine bequeme, sichere und zuverlässige Handhabe ermöglicht, soll erfindungsgemäß die Handhabe um mindestens drei Freiheitsgrade gegenüber dem Instrument verstellbar am Instrumentenschaft festlegbar sein. Zwar ermöglicht diese Ausgestaltung die Adaption relativ zum Instrumentenschaft, eine genaue Adaption an die Bedürfnisse des Anwenders ist jedoch auch hier nicht ausreichend gegeben.

US 6,540,737 zeigt ein Griffteil für ein chirurgisches Instrument, der relativ zu einem Instrumentenschaft verschwenk- und in unterschiedlichen Schwenkpositionen arretierbar ist. Auch dieses Instrument erfüllt jedoch die Anforderungen an die Anpassbarkeit nicht zufriedenstellend.

Die bisher bekannten Griffteil-Ausführungen bieten dem Chirurgen zum Teil durch eine spezielle Formgebung und Anordnung der Griffteile zwar die Möglichkeit, verschiedene Greifstellungen an dem Griffteil einzunehmen. Trotzdem ist eine bequeme Hand- und Armstellung nicht immer möglich. Insbesondere bei begrenzten Platzverhältnissen und einer schwer zugänglichen Operationsstelle ist eine abgewinkelte, ermüdende Handstellung oftmals unvermeidbar. Bei längeren Operationen kann dies dazu führen, dass ein entspanntes Halten und zielgenaues Positionieren nicht mehr möglich ist, wodurch das Operationsergebnis nachteilig beeinflusst wird.

Demgemäss liegt der vorliegenden Erfindung die Aufgabe zugrunde, ein Griffteil für chirurgische Instrumente bereitzustellen, welches die oben genannten Nachteile nicht aufweist und insbesondere eine bequeme Hand- und Armstellung des Chirurgen ermöglicht.

Diese Aufgabenstellung wird durch ein Griffteil nach Anspruch 1 gelöst. Vorteilhafte Ausfiihrungsformen, sowie ein erfindungsgemäßes chirurgisches Instrument und eine vorteilhafte Verwendung eines erfindungsgemäßen Griffteils, sind den weiteren Ansprüchen zu entnehmen.

Der Erfindung liegt der wesentliche Gedanke zugrunde, ein Griffteil zu konzipieren, welches an die jeweiligen Gegebenheiten einer Operation, insbesondere an die räumliche Anordnung zwischen der Operationsstelle und dem Chirurgen, anpassbar ist. Dieser Gedanke wird dadurch umgesetzt, dass das Griffteil einen ersten Teil aufweist, der mit dem chirurgischen Instrument verbindbar oder verbunden ist, und einen zweiten Teil aufweist, der zum Greifen ausgebildet ist und der relativ zum ersten Teil bewegbar ist. Demgemäss ist an dem Griffteil ein Handgriff vorgesehen, der relativ zu einer Instrumentenhalterung, die an das chirurgische Instrument angepasst ist, drehbar und/oder verschiebbar ist.

Das erfindungsgemäße Griffteil ermöglicht also, die Stellung zwischen dem Handgriff und der Instrumentenhalterung, und demgemäss die Stellung zwischen dem Handgriff und dem chirurgischem Instrument, an die gegebene räumliche Anordnung anzupassen. Die vor Ort durchführbare Ausrichtung des Handgriffs ermöglicht eine ergonomische Handstellung, selbst wenn ein Arbeitsende des chirurgischen Instruments an seitliche und/oder schlecht zugängliche Operationsstellen zu führen ist.

Das Griffteil ist für eine Vielzahl von chirurgischen Instrumenten, insbesondere jedoch für Rohrschaftinstrumente, verwendbar, unabhängig davon, welches Werkzeug daran angeordnet ist und wie es zu betätigen ist. Auch bei der Gestaltung des Handgriffs und der Instrumentenhalterung können bekannte und bewährte Formengebungen und Anordnungen berücksichtigt werden, sofern sie eine Drehung und/oder Verschiebung des Handgriffs relativ zu der Instrumentenhalterung zulassen.

Mitunter kann der Chirurg, beispielsweise durch weitere Geräte am Operationstisch, gehindert werden, seine Hand so hinter dem sich länglich erstreckenden chirurgischen Instrument zu positionieren, dass er das chirurgische Instrument in der gewünschten Ausrichtung an die Operationsstelle führen kann. Diesem Problem wird abgeholfen, indem der Handgriff relativ zu der Instrumentenhalterung entlang einer Verschiebungslinie, die senkrecht zu der Längsrichtung des chirurgischen Instruments verläuft, verschiebbar ist. Die Verschiebungslinie ist senkrecht zu der Griffteil-Ebene angeordnet.

Das Griffteil ermöglicht vorzugsweise, dass das chirurgische Instrument auch in einer seitlich zu dem Unterarm des Chirurgen abgewinkelten Ausrichtung haltbar ist, ohne dass dazu das Handgelenk seitlich abgeknickt werden muss. Für die genannten Richtungsbezeichnungen wird davon ausgegangen, dass eine Griffteil-Ebene, in welcher sich der längliche Handgriff und die Instrumentenhalterung vorwiegend erstrecken, vertikal angeordnet ist. Ferner ist bei der Bezugnahme auf die Ausrichtung des chirurgischen Instruments zu berücksichtigen, dass für das Griffteil geeignete chirurgische Instrumente vorzugsweise länglich ausgebildet sind und mit einem proximalen Ende mit der Instrumentenhalterung verbunden sind. Dementsprechend ist ihre Ausrichtung durch die Orientierung ihrer Längsrichtung gegeben. Um ein seitliches Abknicken des Handgelenks zu vermeiden, ist der Handgriff um eine Handgriff-Drehachse, die in der Griffteil-Ebene und im Wesentlichen parallel zu der Längsrichtung des Handgriffs verläuft, drehbar. Die Handgriff-Drehachse ist vorzugsweise im Wesentlichen zentral in dem Handgriff angeordnet, und verläuft durch eine Verbindungsstelle des Griffteils, in welcher der längliche Handgriff und die Instrumentenhalterung zusammenführen. Der längliche Handgriff ist vorzugsweise derart geformt, dass er insgesamt oder nur zum Teil umgreifbar oder eingreifbar ist.

Häufig tritt der Fall auf, dass das chirurgische Instrument nach unten oder nach oben geneigt gehalten werden muss. Für die Richtungsbezeichnungen wird wiederum davon ausgegangen, dass die Griffteil-Ebene vertikal angeordnet ist. Um auch hierbei ein Abknicken des Handgelenks zu vermeiden, ist der Handgriff um eine Schwenkachse, die im Wesentlichen senkrecht zu der Griffteil-Ebene verläuft, schwenkbar. Demgemäss kann durch diese Anordnung der Winkel, der zwischen dem Handgriff und der Instrumentenhalterung eingeschlossen wird, verändert werden, und somit auch eine Neigung des chirurgischen Instruments nach oben oder unten ausgeglichen werden.
Gemäß einer bevorzugten Ausführungsform ist der Handgriff um bis zu 60°, insbesondere um bis zu 45°, aus einer Grundstellung um die Handgriff-Drehachse drehbar. Vorzugsweise ist der Handgriff in beiden Drehrichtungen um diesen Winkel aus der Grundstellung drehbar.

Vorzugsweise ist eine Grundstellung vorgesehen, in welcher der Handgriff in der Griffteil-Ebene angeordnet ist Gemäß einer bevorzugten Ausführungsform ist der Handgriff aus der Grundstellung entlang der Verschiebungslinie um bis zu 10 mm verschiebbar. Insbesondere kann vorgesehen sein, dass der Handgriff aus der Grundstellung in beiden Richtungen entlang der Verschiebungslinie um diese Strecke verschiebbar ist.

Vorzugsweise ist das Griffteil derart ausgelegt, dass verschiedene chirurgische Instrumente darin einbaubar sind. Insbesondere verschiedene Rohrschaftinstrumente mit verschiedenen Werkzeugen an ihrem distalen Ende können dann einfach ausgetauscht werden. Eine dieser Anforderung entsprechende Ausführungsform der Erfindung weist in der Instrumentenhalterung eine Aufnahme auf, in welcher ein proximaler Endabschnitt eines chirurgischen Instruments befestigbar ist

Weiterhin vorteilhaft ist, wenn unterschiedliche Orientierungen des Werkzeuges des chirurgischen Instruments relativ zu dem Griffteil einstellbar sind. Demgemäss ist die Aufnahme derart auszubilden, dass das chirurgische Instrument in verschiedenen Orientierungen in der Aufnahme befestigbar ist Darunter wird insbesondere verstanden, dass das chirurgische Instrument mit seinem proximalen Endabschnitt in verschiedenen Drehstellungen bezüglich einer Drehachse, die der Längsrichtung des chirurgischen Instruments entspricht, in der Aufnahme befestigbar ist

Ferner kann das Griffteil auch nur für ein bestimmtes chirurgisches Instrument vorgesehen sein. Demgemäss kann die Instrumentenhalterung integral mit dem chirurgischen Instrument ausgebildet sein. Insbesondere wenn die Instrumentenhalterung einteilig mit einem Teil des chirurgischen Instruments gebildet ist, ist eine Trennung des Griffteils von dem chirurgischen Instrument und dementsprechend die Verwendung eines Griffteils für verschiedene chirurgische Instrumente nicht möglich. Vorteilhaft an einer solchen Anordnung ist, dass eine verhältnismäßig kompakte Bauweise des chirurgischen Instruments erzielbar ist, was ein Reinigen desselben erleichtert.

Da durch Kraftausübung auf den Handgriff das chirurgische Instrument an eine gewünschte Operationsstelle positionierbar sein soll, sind an dem Griffteil vorzugsweise Arretiermittel vorgesehen, durch welche der Handgriff in einer Grundstellung und mindestens in einer gedrehten, verschobenen und/oder herausgeschwenkten Stellung relativ zu der Instrumentenhalterung, und damit auch relativ zu dem chirurgischen Instrument feststellbar ist Dadurch wird gewährleistet, dass die Bewegungen, die an dem Handgriff durchgeführt werden, auch auf das distale Ende des chirurgischen Instruments übertragen werden. Vorzugsweise ist der Handgriff in jeder Stellung, die er relativ zu der Instrumentenhalterung einnehmen kann, durch die Arretiermittel feststellbar.

Wie bereits diskutiert wurde, weisen chirurgische Instrumente an ihrem distalen Ende oftmals ein Werkzeug auf, das durch Betätigen eines Betätigungselements bewegbar ist oder an welches durch Betätigen eines Betätigungselements elektrische Spannung anlegbar ist Solch ein Betätigungselement ist vorzugsweise an der Instrumentenhalterung und/oder dem Handgriff angeordnet. Ist das Betätigungselement an dem Handgriff angeordnet, so ist der Übertragungsmechanismus, welcher die ausgelöste Bewegung oder das Signal zu dem Werkzeug am distalen Ende des chirurgischen Instruments überträgt, derart auszulegen, dass er ein Drehen und/oder Verschieben des Handgriffs zulässt Vorzugsweise ist das Betätigungselement als Schwenkhebel ausgebildet Ein Betätigen erfolgt beispielsweise durch ein Heranziehen des Schwenkhebels in Richtung des Handgriffs.

Die genannten erfindungsgemäßen Vorteile ergeben sich auch für ein chirurgisches Instrument, insbesondere für ein chirurgisches Rohrschaftinstrument, welches ein erfindungsgemäßes Griffteil aufweist Eine bevorzugte Anordnung eines chirurgischen Instruments ergibt sich, wenn das chirurgische Instrument länglich ausgebildet ist, am distalen Ende ein Werkzeug und am proximalen Ende das Griffteil aufweist, und ferner die Längsrichtung des chirurgischen Instruments im Wesentlichen in der Griffteil-Ebene verläuft Insbesondere ist vorteilhaft, wenn sich der Handgriff abgewinkelt zu der Längsrichtung des chirurgischen Instruments von der Instrumentenhalterung weg erstreckt.

Vorteilhaft ist auch die Verwendung eines erfindungsgemäßen Griffteils zum Halten eines chirurgischen Instruments, insbesondere zum Halten eines chirurgischen Rohrschaftinstruments.

Vorteile und Zweckmäßigkeiten ergeben sich im Übrigen aus der nachfolgenden Beschreibung bevorzugter Ausführongsbeispiele anhand der Figuren. Von diesen zeigen:
- Fig. 1 eine perspektivische Ansicht eines etfindungsgemäßen Griffteils;
- Fig. 2 eine Draufsicht von oben auf das Griffteil von Fig. 1;
- Fig. 3 eine schematische Seitenansicht einer weiteren Ausführungsform eines erfindungsgemäßen Griffteils mit darin gehaltenem Rohrschaftinstrument

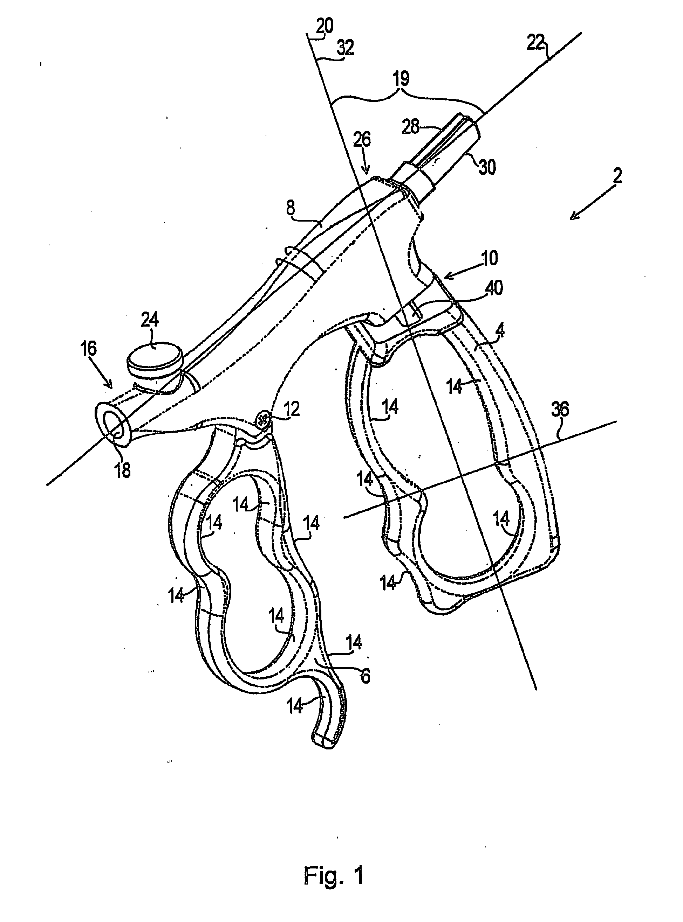

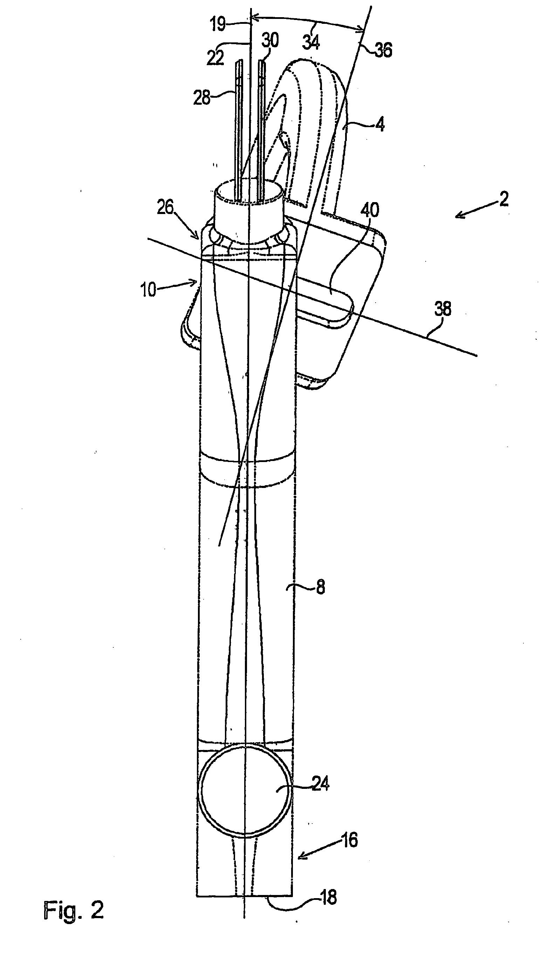

Die Figuren 1 und 2 zeigen ein erfindungsgemäßes Griffteil 2 mit einem Handgriff 4, einem Betätigungselement 6 und einer Instrumentenhalterung 8. Der Handgriff 4 und die Instrumentenhalterung 8 führen in einer Verbindungsstelle 10 zusammen. Das Betätigungselement 6 ist um eine Drehachse 12 schwenkbar an der Instrumentenhalterung 8 angebracht. Feiner sind an dem Handgriff 4 und an dem Betätigungselement 6 eine Vielzahl von Anlageflächen 14 für Handpartien eines Chirurgen vorgesehen, so dass verschiedene Greif- und Bedienstellungen an dem Handgriff 4 und dem Betätigungselement 6 möglich sind.

Die Instrumentenhalterung 8 weist an einem distalen Ende 16 eine Schaftaufnahme 18 auf, in welche ein proximaler Endabschnitt eines chirurgischen Instruments einführbar ist Die gezeigte Schaftaufnahme 18 ist zum Halten eines Rohrschaftes ausgebildet, wobei das proximale Ende des Rohrschaftes in verschiedenen Orientierungen in der Schaftaufnahme 18 befestigbar ist Demgemäß kann ein an dem distalen Ende des Rohrschahinstruments angeordnetes Werkzeug in verschiedenen Winkelstellungen relativ zu einer Griffteil-Ebene 19, welche in einer Grundstellung des Handgriffs 4 durch eine Längsrichtung 20 des Handgriffs 4 und eine Erstreckungsrichtung 22 der Instrumentenhalterung 8 definiert ist, ausgerichtet und befestigt werden. Die Grundstellung des Handgriffs 4 zeichnet sich dadurch aus, dass sich die Längsrichtung 20 des Handgriffs 4 und die Erstreckungsrichtung 22 der Instrumentenhalterung 8 schneiden und nicht quer zueinander verschoben sind. Zum Befestigen des Rohrschaftes in der Schaftaufnahme 18 ist eine Arretieteinrichtung 24 an der Schaftaufnahme 18 vorgesehen, mittels welcher der Innendurchmesser der Schaftaufnahme 18 verkleinerbar ist Durch Arretieren mittels Federkraft der Arretiereinrichtung 24 wird der Rohrschaft in der Schaftaufnahme 18 eingeklemmt.

Ferner ist in der Insttumentenhaltetung 8 ein (nicht gezeigter) Übertragungsmechanismus vorgesehen, durch welchen eine Bewegung des Betätigungselements 6 an ein Werkzeug eines in der Schaftaufnahme 18 gehaltenen Rohrschaftinstrumentes übertragbar ist Durch Ziehen an dem Betätigungselement 6 ist also eine Aktion eines Werkzeuges an einem in der Schaftaufnahme 18 gehaltenen Rohtschaftinstrument bewirkbar.

An einem proximalen Ende 26 der Instrumentenhalterung 8 sind zwei elektrische Kontakte 28, 30 angebracht Ferner sind (nicht gezeigte) elektrische Leitungen, die innerhalb der Instrumentenhalterung 8 verlaufen, von den Kontakten 28, 30 zu zwei entsprechenden Kontakten in der Schaftaufnahme 18 geführt, so dass an ein entsprechend ausgebildetes Rohrschaftinstrument eine elektrische Spannung anlegbar ist Insbesondere kann zwischen zwei Werkzeugteilen eines entsprechenden Rohrschaftinstruments eine hochfrequente Wechselspannung angelegt werden.
Ein geeignetes Rohrschaftinstrument zum Anlegen einer hochfrequenten Wechselspannung ist beispielsweise eine bipolare Zange, bei welcher im eingebauten Zustand die beiden Zangenbacken jeweils leitend mit den elektrischen Kontakten 28, 30 verbunden sind.

Im Folgenden wird mit Bezugnahme auf die Figuren 1 und 2 auf den Handgriff 4 eingegangen, der erfindungsgemäß in verschiedenen Stellungen relativ zu der Instrumentenhalterung 8 positionierbar ist. In den Figuren 1 und 2 ist der Handgriff 4 in einer aus der Griffteil-Ebene 19 herausgeschobenen und gedrehten Stellung gezeigt. Der Handgriff 4 ist um eine Handgriff-Drehachse 32, die in der Grundstellung des Handgriffs 4 der Längsrichtung 20 des Handgriffs 4 entspricht, relativ zu der Instrumentenhalterung 8 drehbar. Wie in Fig. 2 ersichtlich, ist dementsprechend der Winkel 34 zwischen einer Handgriff-Querrichung 36 und der Erstreckungsrichtung 22 der Instrumentenhalterung 8 variierbar.

In einer (nicht gezeigten) Grundstellung verläuft die Handgriff-Längsrichtung 20 und die Handgriff-Querrichtung 36 innerhalb der Griffteil-Ebene 19. Senkrecht zu der Handgriff-Querrichtung 36, und in der Grundstellung des Handgriffs 4 senkrecht zur Griffteil-Ebene 19, verläuft eine Verschiebungslinie 38, entlang welcher der Handgriff 4 relativ zu der Instrumentenhalterung 8 verschiebbar ist. Wird der Handgriff 4 entlang der Verschiebungslinie 38 aus der Grundstellung herausgeschoben, so erstreckt sich der Handgriff 4 und insbesondere dessen Längsrichtung 20 seitlich von der Griffteil-Ebene 19.

Die beschriebene Drehung und Verschiebung des Handgriffs 4 wird durch einen (nicht gezeigten) Stift, der an der Instrumentenhalterung 8 angeordnet ist, und eine an dem Handgriff 4 ausgebildete Aufnahmeöffnung 40 realisiert Der Stift erstreckt sich in der Griffteil-Ebene 19 und senkrecht zu der Erstreckungsrichtung 22 von der Instrumentenhalterung 8 weg durch die Aufnahmeöffnung 40 des Handgriffs 4. Die Aufnahmeöffnung 40 ist länglich und erstreckt sich entlang der Verschiebungslinie 38, so dass der Stift in der Aufnahmeöffnung 40 drehbar und entlang der Verschiebungslinie 38 verschiebbar gehalten ist Der Stift weist ferner eine Verdickung an einem die Aufnahmeöffnung 40 hintergreifenden Abschnitt auf, so dass ein Herausziehen des Handgriffs 4, beispielsweise entlang der Längsrichtung 20 des Handgriffs 4, verhindert wird.

Ferner sind an der Verbindungsstelle 10, in welcher der Stift in die Aufnahmeöffnung 40 führt, (nicht gezeigte) Arretiermittel vorgesehen, mittels welcher eine gewünschte Stellung des Handgriffs 4 relativ zu der Instrumentenhalterung 8 feststellbar ist. Ausgehend von der Grundstellung lässt das gezeigte Griffteil in beiden Drehrichtungen ein Verdrehen des Handgriffs 4 um die Handgriff-Drehachse 32 vorzugsweise um bis zu 60° zu. Ferner wird ein Verschieben des Handgriffs 4 entlang der Verschiebungslinie 38, von der Grundstellung des Handgriffs 4 aus, in beiden Richtungen vorzugsweise um bis zu 10 mm ermöglicht.

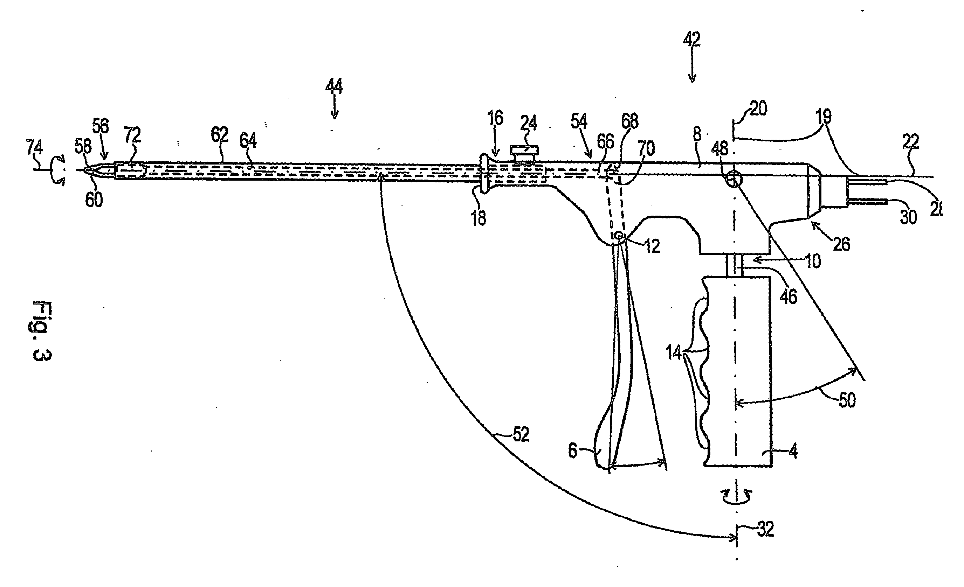

Fig. 3 zeigt schematisch eine weitere Ausführungsform eines erfindungsgemäßen Griffteils 42 mit darin eingebautem Rohrschaftinstrument 44, das als Zange ausgebildet ist Ähnlich zu der ersten Ausführungsform weist auch dieses Griffteil einen Handgriff 4, ein Betätigungselement 6, eine Instrumentenhalterung 8, elektrische Kontakte 28, 30, eine Schaftaufnahme 18 und eine Feststellschraube 24 auf. Auch bei dem in Fig. 3 gezeigten Griffteil 42 erstreckt sich ein Stift 46 von der Instrumentenhalterung 8 in eine (nicht gezeigte) Aufnahmeöffnung des Handgriffs 4, so dass der Handgriff wie in der ersten Ausführungsform relativ zu der Instrumentenhalterung 8 um eine Handgriff-Drehachse 32 drehbar und quer zu einer Griffteil-Ebene 19 verschiebbar ist

Der Stift 46 wird in der Instrumentenhalterung 8 gehalten, wobei er um eine Schwenkachse 48, die senkrecht zu der Griffteil-Ebene 19 verläuft, schwenkbar ist Der Handgriff 4 ist entlang der Pfeilrichtung 50 aus einer Grundposition heraus, in welcher der zwischen Längsrichtung 20 des Handgriffs 4 und Erstreckungsrichtung 22 der Instrumentenhalterung 8 eingeschlossene Winkel 52 90° beträgt, in der Griffteil-Ebene 19 schwenkbar. Gemäß dieser Ausführungsform ist also zusätzlich der zwischen Handgriff 4 und Instrumentenhalterung 8 eingeschlossene Winkel 52 variierbar.

In Fig. 3 ist schematisch ein Übertragungsmechanismus 54 gezeigt, durch welchen eine Bewegung des Betätigungselements 6 auf ein Werkzeug 56, das im gezeigten Fall aus zwei Zangenbacken 58, 60 gebildet wird, übertragbar ist Innerhalb eines Rohrschaftes 62 des Rohrschaftinstrumentes 44 ist eine Schub- und Zugstange 64 angeordnet, deren proximales Ende 66 mittels eines Kugelschnellverschlusses 68 mit einem oberen Abschnitt 70 des Betätigungselements 6 verbindbar ist Durch Betätigen des Betätigungselements 6 wird die Schub- und Zugstange 64 relativ zu dem Rohrschaft 62 verschoben. Ferner ist nahe dem Werkzeug 56 innerhalb des Rohrschaftes 62 eine Umsetzungseinrichtung 72 angeordnet, durch welche die Relativbewegung zwischen Rohrschaft 62 und Schub- und Zugstange 64 in eine Relativbewegung zwischen den Zangenbacken 58, 60 umgesetzt wird, so dass durch Bewegen des Betätigungselements 6 ein Öffnen bzw. Schließen der Zangenbacken 58, 60 bewirkbar ist.

Das Rohrschaftinstrument 44 ist ferner um dessen Längsrichtung 74, die parallel zu der Erstreckungsrichtung 22 der Instrumentenhalterung 8 verläuft, in der Schaftaufnahme 18 drehbar und kann in einer gewünschten Ausrichtung durch Zudrehen der Feststellschraube 24 arretiert werden. Demgemäss kann die Ausrichtung des Werkzeuges 56 relativ zu der Griffteil-Ebene 19 der jeweiligen Anwendung angepasst werden.

Die Erfindung ist nicht auf die beispielhaft gezeigten Ausführungsformen der Figuren 1 bis 3 beschränkt Die Erfindung ergibt sich vielmehr aus einer fachmännischen Gesamtbetrachtung der Ansprüche, der Beschreibung, der beispielhaften Ausführungsfotmen, und der nachfolgend erwähnen Varianten, die einem Fachmann Hinweise auf weitere alternative Ausführungsformen geben sollen.

Insbesondere können die Handgriff-Drehachse, die Verschiebungslinie, die Längsrichtung des Handgriffs, die Erstreckungsrichtung der Instrumentenhalterung und die Schwenkachse auch andere Ausrichtungen zueinander, als sie in den Figuren 1 bis 3 gezeigt sind, aufweisen. Ferner können noch zusätzliche Drehachsen und/oder Verschiebungslinien vorgesehen sein, durch welche weitere Bewegungen des Handgriffs relativ zu der Instrumentenhaltetung zugelassen werden.

Die Verbindung zwischen dem Handgriff und der Instrumentenhalterung, welche ein Drehen und Verschieben des Handgriffs relativ zu der Instrumentenhalterung zulässt, ist nicht auf die gezeigte Anordnung aus Stift und Aufnahmeöffnung begrenzt. Insbesondere kann auch der Handgriff einen Stift und die Instrumentenhalterung eine Aufnahmeöffnung für den Stift aufweisen. Ferner kann auch ein Kugelgelenk zwischen Handgriff und Instrumentenhalterung vorgesehen sein, welches zumindest eine Drehbewegung des Handgriffs um eine Handgriff-Drehachse, die im Wesentlichen innerhalb der Griffteil-Ebene verläuft, und eine Schwenkbewegung des Handgriffs um eine Schwenkachse, die quer zur Griffteil-Ebene verläuft, zulässt.

Zur Übertragung einer Bewegung des Betätigungselements auf ein Werkzeug des chirurgischen Instruments ist neben der gezeigten Anordnung aus Rohrschaft und relativ dazu beweglicher Schub- und Zugstange, die mechanisch mit dem Betätigungselement verbindbar oder verbunden ist, noch eine Vielzahl weiterer Übertragungsmechanismen bekannt, die gleichfalls in einem erfindungsgemäßen Griffteil umsetzbar sind.

Die gezeigte Formgebung des Handgriffs, des Betätigungselements und der Instrumentenhalterung ist für die Ausführung der Erfindung nicht entscheidend, so dass hierbei weitere Variationsmöglichkeiten bestehen. Insbesondere das Betätigungselement kann gleichermaßen als Schiebeschalter, Drückschalter o.ä. ausgebildet sein. Auch für die Anbringung der elektrischen Kontakte ergeben sich weitete Variationsmöglichkeiten.

Ferner ist in den Figuren 1 bis 3 eine Schaftaufnahme gezeigt, welche zur Aufnahme eines Rohrabschnitts eines Rohrschaftinstrumentes ausgebildet ist An der Instrumentenhaltetung kann jedoch auch eine anderweitige Aufnahme vorgesehen sein, in welcher ein proximaler Endabschnitt eines chirurgischen Instrument befestigbar ist Auch die Mittel zum Befestigen des chirurgischen Instruments in der Aufnahme müssen nicht als Feststellschraube realisiert sein, sondern können in Form eines anderweitigen Klemm- oder Feststellmittels ausgebildet sein.

### Bezugszeichenliste

- 2: Griffteil
- 4: Handgriff
- 6: Betätigungselement
- 8: Instrumentenhalterung
- 10: Verbindungsstelle
- 12: Drehachse
- 14: Anlagefläche
- 16: distales Ende
- 18: Schaftaufnahme
- 19: Griffteil-Ebene
- 20: Längsrichtung (des Handgriffs)
- 22: Erstreckungsrichtung (der Instrumentenhalterung)
- 24: Feststellschraube
- 26: proximales Ende
- 28,30: elektrischer Kontakte
- 32: Handgriff-Drehachse
- 34: Winkel
- 36: Handgriff-Querrichtung
- 38: Verschiebungslinie
- 40: Aufnahmeöffnung
- 42: Griffteil
- 44: Rohrschaftinstrument
- 46: Stift
- 48: Schwenkachse
- 50: Pfeilrichtung
- 52: Winkel
- 54: Übertragungsmechanismus
- 56: Werkzeug
- 58, 60: Zangenbacken
- 62: Rohrschaft
- 64: Schub- und Zugstange
- 66: proximales Ende
- 68: Kugelschnellverschluss
- 70: oberer Abschnitt
- 72: Umsetzungseinrichtung
- 74: Längsrichtung

## Patentansprüche

1. Griffteil für ein chirurgisches Instrument, insbesondere für ein chirurgisches Rohrschaftinstrument (44), aufweisend:
einen Handgriff (4) und eine an das chirurgische Instrument angepasste Instrumentenhalterung (8), wobei
der Handgriff relativ zu der Instrumentenhalterung drehbar und/oder verschiebbar ist,
wobei sich der Handgriff (4) und die Instrumentenhalterung (8) vorwiegend in einer Griffteil-Ebene (19), erstrecken, und wobei der Handgriff relativ zu der Instrumenten-halterung entlang einer im Wesentlichen senkrecht zu der Griffteil-Ebene verlaufender Verschiebunglinie (38) verschiebbar ist.

2. Griffteil nach Anspruch 1,
**gekennzeichnet durch**
eine Griffteil-Ebene (19), in welcher sich der längliche Handgriff (4) und die Instrumentenhalterung (8) vorwiegend erstrecken, und **durch** eine Handgriff-Drehachse (32), die in der Griffteil-Ebene und im Wesentlichen parallel zu der Längsrichtung (20) des Handgriffs verläuft, wobei der Handgriff um die Handgriff-Drehachse drehbar ist.

3. Griffteil nach einem der vorangehenden Ansprüche,
**dadurch gekennzeichnet, dass**
der Handgriff (4) in einer Griffteil-Ebene (19), entlang welcher sich der Handgriff und die Instrumentenhalterung (8) vorwiegend erstrecken, um eine Schwenkachse (48), die im Wesentlichen senkrecht zu der Griffteil-Ebene verläuft, schwenkbar ist.

4. Griffteil nach einem der Ansprüche 2 oder 3,
**dadurch gekennzeichnet, dass**
der Handgriff (4) um die Handgriff-Drehachse (32), insbesondere in beiden Drehrichtungen, um bis zu 60° aus einer Grundstellung heraus drehbar ist.

5. Griffteil nach einem der Ansprüche 1 bis 4,
**dadurch gekennzeichnet, dass**
der Handgriff (4) aus einer Grundstellung, in welcher der Handgriff in der Griffteil-Ebene (19) angeordnet ist, entlang der Verschiebungslinie (38), insbesondere in beiden Richtungen, um bis zu 10 mm verschiebbar ist.

6. Griffteil nach einem der vorangehenden Ansprüche,
**dadurch gekennzeichnet, dass**
die Instrumentenhalterung (8) eine Aufnahme (18) aufweist, in welcher ein proximaler Endabschnitt eines chirurgischen Instruments, insbesondere in verschiedenen Orientierungen, befestigbar ist.

7. Griffteil nach einem der Ansprüche 1 bis 5,
**dadurch gekennzeichnet, dass**
die Instrumentenhalterung (8) integral mit dem chirurgischen Instrument, insbesondere einteilig mit einem Teil des chirurgischen Instruments, ausgebildet ist.

8. Griffteil nach einem der vorangehenden Ansprüche,
**dadurch gekennzeichnet, dass**
Arretiermittel vorgesehen sind, durch welche der Handgriff (4) in einer Grundstellung und mindestens in einer gedrehten, verschobenen und/oder herausgeschwenkten Stellung relativ zu der Instrumentenhalterung (8) feststellbar ist.

9. Griffteil nach einem der vorangehenden Ansprüche,
**dadurch gekennzeichnet, dass**
die Instrumentenhalterung (8) und/oder der Handgriff (4) ein Betätigungselement (6) zum Betätigen des chirurgischen Instruments aufweist.

10. Griffteil nach Anspruch 9,
**dadurch gekennzeichnet, dass**
das Betätigungselement (6) als Schwenkhebel ausgebildet ist.

11. Chirurgisches Instrument, insbesondere chirurgisches Rohrschaftinstrument (44), mit einem Griffteil (2;42) nach einem der vorangehenden Ansprüche.

12. Chirurgisches Instrument, insbesondere chirurgisches Rohrschaftinstrument (44), nach Anspruch 11,
**dadurch gekennzeichnet, dass**
das chirurgische Instrument länglich ausgebildet ist, dass an dessen distalen Ende ein Werkzeug (56) und an dessen proximalen Ende das Griffteil (2;42) angeordnet ist, wobei die Längsrichtung (74) des chirurgischen Instruments im Wesentlichen in einer Griffteil-Ebene (19), in welcher sich der längliche Handgriff (4) und die Instrumentenhalterung (8) vorwiegend erstrecken, verläuft, und wobei sich der Handgriff abgewinkelt zu der Längsrichtung des chirurgischen Instruments von der Instrumentenhalterung weg erstreckt.

13. Verwendung eines Griffteils nach einem der Ansprüche 1 bis 10 zum Halten eines chirurgischen Instruments, insbesondere zum Halten eines chirurgischen Rohrschaftinstruments (44).

## Claims

1. Grip part for a surgical instrument, especially for a tubular-shaft surgical instrument (44), comprising:
a hand-grip (4), and an instrument holder (8) matching the surgical instrument, wherein
the hand-grip is rotatable and/or displaceable relative to the instrument holder, wherein the hand-grip (4) and the instrument holder (8) extend mainly in a grip-part plane (19), and wherein the hand-grip is displaceable relative to the instrument holder along a displacement line (38) running substantially perpendicular to the grip-part plane.

2. Grip part according to claim 1,
**characterised by**
a grip-part plane (19) in which the elongate hand-grip (4) and the instrument holder (8) mainly extend, and by a hand-grip axis of rotation (32) which runs in the grip-part plane and substantially parallel to the longitudinal direction (20) of the hand-grip, the hand-grip being rotatable about the hand-grip axis of rotation.

3. Grip part according to one of the preceding claims,
**characterised in that**
the hand-grip (4) is pivotable in a grip-part plane (19), along which the hand-grip and the instrument holder (8) mainly extend, about a pivot axis (48), which runs substantially perpendicular to the grip-part plane.

4. Grip part according to one of claims 2 or 3,
**characterised in that**
the hand-grip (4) is rotatable about the hand-grip axis of rotation (32), especially in both directions of rotation, by up to 60° out of a base position.

5. Grip part according to one of claims 1 to 4,
**characterised in that**
the hand-grip (4) is displaceable from a base position, in which the hand-grip is arranged in the grip-part plane (19), along the displacement line (38), especially in both directions, by up to 10 mm.

6. Grip part according to one of the preceding claims,
**characterised in that**
the instrument holder (8) has a seating (18) in which a proximal end portion of a surgical instrument is fastenable, especially in various orientations.

7. Grip part according to one of claims 1 to 5,
**characterised in that**
the instrument holder (8) is of integral construction with the surgical instrument, especially of one-piece construction with a part of the surgical instrument.

8. Grip part according to one of the preceding claims,
**characterised in that**
locking means are provided by means of which the hand-grip (4) is fixable in a base position and in at least one rotated, displaced and/or pivoted-out position relative to the instrument holder (8).

9. Grip part according to one of the preceding claims,
**characterised in that**
the instrument holder (8) and/or the hand-grip (4) has an actuating element (6) for actuation of the surgical instrument.

10. Grip part according to claim 9,
**characterised in that**
the actuating element (6) is in the form of a pivoting lever.

11. Surgical instrument, especially a tubular-shaft surgical instrument (44), having a grip part (2; 42) according to one of the preceding claims.

12. Surgical instrument, especially a tubular-shaft surgical instrument (44), according to claim 11,
**characterised in that**
the surgical instrument is of elongate construction, and **in that** at the distal end thereof there is arranged a tool (56) and at the proximal end thereof there is arranged the grip part (2; 42), the longitudinal direction (74) of the surgical instrument running substantially in a grip-part plane (19), in which the elongate hand-grip (4) and the instrument holder (8) mainly extend, and the hand-grip extending away from the instrument holder at an angle to the longitudinal direction of the surgical instrument.

13. Use of a grip part according to one of claims 1 to 10 in holding a surgical instrument, especially in holding a tubular-shaft surgical instrument (44).

## Revendications

1. Partie de préhension d'un instrument chirurgical, notamment d'un instrument chirurgical à tige tubulaire (44), comprenant :
une poignée (4) et un porte-instrument (8) adapté à l'instrument chirurgical,
la poignée étant mobile en rotation et/ou en translation par rapport au porte-instrument,
la poignée (4) et le porte-instrument (8) s'étendant principalement dans un plan de la partie de préhension (19) et la poignée étant mobile en translation par rapport au porte-instrument le long d'une ligne de translation (38) sensiblement perpendiculaire au plan de la partie de préhension.

2. Partie de préhension selon la revendication 1, **caractérisée par** un plan de la partie de préhension (19) dans lequel la poignée oblongue (4) et le porte-instrument (8) s'étendent principalement, et
**caractérisée par** un axe de rotation (32) de la poignée situé dans le plan de la partie de préhension et sensiblement parallèle à la direction longitudinale (20) de la poignée, ladite poignée étant mobile en rotation autour de cet axe de rotation de la poignée.

3. Partie de préhension selon l'une quelconque des revendications précédentes, **caractérisée en ce que** la poignée (4) est apte à pivoter, dans un plan de la partie de préhension (19) dans lequel la poignée et le porte-instrument (8) s'étendent principalement, autour d'un axe de pivotement (48) sensiblement perpendiculaire au plan de la partie de préhension.

4. Partie de préhension selon la revendication 2 ou la revendication 3, **caractérisée en ce que** la poignée (4) est mobile en rotation, notamment dans les deux sens, autour de l'axe de rotation (32) de la poignée, sur une amplitude angulaire de 60° maximum de part et d'autre d'une position de base.

5. Partie de préhension selon l'une quelconque des revendications 1 à 4, **caractérisée en ce que** la poignée (4), à partir d'une position de base dans laquelle elle est comprise dans le plan de la partie de préhension (19), est mobile en translation le long de la ligne de translation (38), notamment dans les deux sens, sur une distance pouvant aller jusqu'à 10 mm.

6. Partie de préhension selon l'une quelconque des revendications précédentes, **caractérisée en ce que** le porte-instrument (8) présente un logement (18) dans lequel peut être fixée une extrémité proximale d'un instrument chirurgical, notamment selon différentes orientations.

7. Partie de préhension selon l'une quelconque des revendications 1 à 5, **caractérisée en ce que** le porte-instrument (8) est conçu d'un seul tenant avec l'instrument chirurgical, notamment monobloc avec une partie de l'instrument chirurgical.

8. Partie de préhension selon l'une quelconque des revendications précédentes, **caractérisée en ce que** sont prévus des moyens d'arrêt permettant de bloquer la poignée (4) dans une position de base et au moins dans une position de rotation, de translation et/ou de pivotement par rapport au porte-instrument (8).

9. Partie de préhension selon l'une quelconque des revendications précédentes, **caractérisée en ce que** le porte-instrument (8) et/ou la poignée (4) présente(nt) un élément d'actionnement (6) destiné à actionner l'instrument chirurgical.

10. Partie de préhension selon la revendication 9, **caractérisée en ce que** l'élément d'actionnement (6) est conçu comme un levier pivotant.

11. Instrument chirurgical, notamment instrument chirurgical à tige tubulaire (44), comportant une partie de préhension (2 ; 42) selon l'une quelconque des revendications précédentes.

12. Instrument chirurgical, notamment instrument chirurgical à tige tubulaire (44) selon la revendication 11, **caractérisé en ce que** l'instrument chirurgical est de forme oblongue, **en ce qu'**un outil (56) est agencé à son extrémité distale et **en ce que** la partie de préhension (2 ; 42) est agencée à son extrémité proximale, la direction longitudinale (74) de l'instrument chirurgical se trouvant sensiblement dans un plan de la partie de préhension (19) dans lequel la poignée oblongue (4) et le porte-instrument (8) s'étendent principalement, et la poignée s'éloignant du porte-instrument en formant un coude avec la direction longitudinale de l'instrument chirurgical.

13. Utilisation d'une partie de préhension selon l'une quelconque des revendications 1 à 10 pour tenir un instrument chirurgical, notamment pour tenir un instrument chirurgical à tige tubulaire (44).
